(19) Europäisches Patentamt
European Patent Office
Office européen des brevets

(11) Veröffentlichungsnummer: **0 193 801**
**B1**

(12) # EUROPÄISCHE PATENTSCHRIFT

(45) Veröffentlichungstag der Patentschrift: **18.01.89**

(21) Anmeldenummer: **86102204.4**

(22) Anmeldetag: **20.02.86**

(51) Int. Cl.⁴: **C 07 C 27/00**, C 07 C 41/50, C 07 C 45/49, C 07 C 47/06, C 07 C 67/36

(54) **Verfahren zur kontinuierlichen Homologisierung von Methanol.**

(30) Priorität: **26.02.85 DE 3506714**

(43) Veröffentlichungstag der Anmeldung: **10.09.86 Patentblatt 86/37**

(45) Bekanntmachung des Hinweises auf die Patenterteilung: **18.01.89 Patentblatt 89/3**

(84) Benannte Vertragsstaaten: **AT BE CH DE FR GB IT LI LU NL SE**

(56) Entgegenhaltungen:
**EP-A- 0 011 042**
**EP-A- 0 037 586**
**EP-A- 0 141 535**
**DE-A- 3 341 907**
**DE-A- 3 343 519**

(73) Patentinhaber: **Union Rheinische Braunkohlen Kraftstoff Aktiengesellschaft, Ludwigshafener Strasse o. Nr. Postfach 8, D-5047 Wesseling (DE)**

(72) Erfinder: **Korff, Joachim, Dr., Kapellenstrasse 28, D-5303 Bornheim-Merten (DE)**
Erfinder: **Keim, Karl-Heinz, Dipl.-Ing., Höhenring 31, D-5351 Heimerzheim (DE)**
Erfinder: **Keim, Wilhelm, Prof. Dr., Brüsseler Ring, D-5100 Aachen (DE)**
Erfinder: **Röper, Michael, Dr., Albert-Schweizer-Strasse 10, D-6706 Wachenheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft ein kontinuierliches Verfahren zur Homologisierung von Methanol in Gegenwart von 1,4-Dioxan(en) mit Homologisierungskatalysatoren, die ohne wesentlichen Aktivitätsverlust über lange Laufzeiten rückführbar sind.

Während des letzten Jahrzehnts sind zahlreiche Katalysatoren bekannt geworden, die in Homologisierungsreaktionen, insbesondere für die Homologisierung von Methanol eingesetzt werden können. Es handelt sich in den überwiegenden Fällen um Mehrkomponenten-Katalysatoren, die aus Kobalt oder Nickel oder deren Verbindungen bestehen, einem salzartigen oder homöopolaren Jodid oder Bromid und einer Vielzahl möglicher Liganden. Hierbei sind insbesondere Stickstoff und Phosphor enthaltende organische Verbindungen zu nennen.

In US-PS 4 190 729 wird die Homologisierung von Methanol zu Ethanol, Acetaldehyd und Methylacetat mit Kobaltcarbonyl und einem Phosphinoxid offenbart, bevorzugt in hochsiedenden Lösungsmitteln wie Naphthalin oder Methylnaphthalin, wobei der Katalysator rückgeführt wird.

Die Ausbeuten und Selektivitäten mit dem offenbarten Katalysator sind jedoch gering.

In EP-A-0 010 373 wird ein Katalysatorsystem für die Homologisierung von Methanol beschrieben aus Kobalt, einem Jodid oder Bromid und einem mehrzähnigen Liganden der allgemeinen Formel

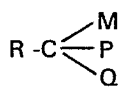

in dem R eine Alkylgruppe, M, P und Q -X $(R_1)(R_2)$, -Y $(R_3)(R_4)$ und -Z $(R_5)(R_6)$ sein können, wobei X,Y und Z Stickstoff, Phosphor, Arsen, Antimon oder Wismuth sein können.

Ein diskontinuierliches Verfahren zur Herstellung von Acetaldehyd aus Methanol und $H_2/CO$ in Gegenwart eines Kobalt und Jod enthaltenden Katalysators ist in EP-A-0 037 586 offenbart. Gemäss diesem Verfahren wird in einem inerten Lösungsmittel, bevorzugt in Dioxan, gearbeitet.

In EP-A-0 011 042 wird ein Katalysator beschrieben für die Herstellung von Acetaldehyd aus Methanol bestehend aus Kobalt, salzartigem Jodid und einem kovalenten Halogenid, wobei das kovalente Halogenid 0,1 bis 10% in Grammatomen des salzartigen Jodids beträgt. Als Kationen im salzartigen Jodid sind Alkali, Erdalkali, Ammonium, quaternäres Ammonium, Phosphonium und quaternäres Phosphonium genannt.

Bevorzugt sind genannt: LiJ, NaJ, KJ, CsJ und $CaJ_2$. Als Gründe für die Bevorzugung sind angegeben: Löslichkeit, Verfügbarkeit und Handhabbarkeit (S. 3, Zeilen 36-38).

In EP-A-0 018 927 wird ein Verfahren zur Herstellung von Essigsäure durch Carbonylieren von Methanol beschrieben unter Verwendung eines Katalysators, der Nickel, ein Alkali- oder Erdalkalihalogenid, und kovalentes Halogenid enthält in den Lösungsmitteln Polyglykoldialkylether, Tetramethylensulfon und Säureamiden. Auf Seite 3, Zeilen 17/18 wird offenbart, dass das erfindungsgemässe System auch die Rückführung der Katalysatorkombination erleichtert.

Nähere Angaben über die Rückführbarkeit sind weder in den Ansprüchen noch in der Beschreibung oder den Beispielen enthalten. Nach Untersuchungen der Anmelderin besitzt Tetramethylensulfon (Sulfolan) keinen die Homologisierung von Methanol begünstigenden Effekt.

Untersuchungen der Anmelderin mit Kobalt-Katalysatoren haben ergeben, dass Katalysatorsysteme zur Umwandlung von Methanol mit Wasserstoff und Kohlenmonoxid, die Alkali- oder Erdalkali-Halogenide sowie homöopolare Halogenide enthalten, in den in der EP-A-0 018 927 offenbarten Lösungsmitteln nämlich Sulfolan, Polyethylenglykol oder Amiden nicht geeignet sind für technische Homologisierungsverfahren, in denen Katalysatoren im Kreislauf geführt werden.

Sowohl mit Alkali-, Ammonium- als auch mit Erdalkalihalogeniden treten nach Untersuchungen der Anmelderin in Gegenwart der offenbarten Lösungsmittel Zersetzungen der letzteren bereits nach 2-3maliger Rückführung auf, begleitet von einem deutlichen Rückgang der Methanol-Umsätze und Produktselektivitäten.

Überraschend und vom Fachmann unvorhersehbar wurde von der Anmelderin ein Verfahren zur Homologisierung von Methanol gefunden, dadurch gekennzeichnet, dass die Homologisierung unter Verwendung des Katalysatorsystems enthaltend:

a) Kobalt und/oder (eine) Kobaltverbindung(en),

b) wenigstens ein Jodid aus der Gruppe LiJ, NaJ, KJ, RbJ, CsJ, $(N\ R_1R_2R_3R_4)^+J^-$, und/oder wenigstens eines dieser Jodide bildende Verbindungen, wobei $R_1$, $R_2$, $R_3$ und $R_4$ H, Alkyl- und Arylgruppen, bevorzugt H, $CH_3$, $C_2H_5$ und $C_6H_5$ sein können,

c) Methyljodid und/oder Methyljodid bildende Verbindungen in Gegenwart von 1,4-Dioxan und/oder alkyliertem 1,4-Dioxan durchgeführt wird und dass die Homologisierung kontinuierlich unter Rückführung des Katalysatorsystems durchgeführt wird. Hierbei treten auch bei langen Laufzeiten keine Zersetzungen, Ausbeute- und Selektivitätsverluste auf.

Das rückgeführte Katalysatorsystem kann Wasser, Essigsäure, höhere Homologisierungsprodukte und Dioxan(e) enthalten.

Erfindungsgemäss muss das Katalysatorsystem Kobalt in feinverteilter, elementarer Form oder in Form seiner Verbindungen wie z.B. Kobaltacetat, Kobaltcarbonylen, Kobaltcarbonylaten, Kobaltjodid, Kobaltbromid und anderen enthalten sowie wenigstens eine der salzartigen Komponenten LiJ, NaJ, KJ, RbJ, CsJ, $(N\ R_1R_2R_3R_4)^+J^-$, wobei $R_1$, $R_2$, $R_3$ und $R_4$ bevorzugt H, $CH_3$, $C_2H_5$ und $C_6H_5$ jedoch auch andere Alkyl- und Arylgruppen sein können. Diese N-Substituenten können einander beliebig ersetzen. Es können anstelle der Jodide oder zusätzlich auch Verbindungen eingesetzt werden, die wenigstens eines der genannten Jodide bilden, wie z.B. Kaliumacetat/HJ oder Kaliumacetat/$CH_3J$. Bevorzugt sind NaJ, KJ und $NH_4^+J^-$ sowie $N(CH_3)_4^+J^-$ bzw. diese Jodide bildende Verbindungen. Das Katalysatorsystem muss zusätzlich Methyljodid enthalten und/oder Methyljodid bildende Verbindungen.

Wird die Homologisierung des Methanols mit diesem Katalysatorsystem in 1,4-Dioxan und/oder alkyliertem, bevorzugt methyliertem oder ethyliertem 1,4-Dioxan, wie z.B. in einzeln oder mehrfach methyliertem oder ethyliertem 1,4-Dioxan oder deren Gemischen durchgeführt, so zeigt sich überraschend, dass im Gegensatz zum Stand der Technik im Hinblick auf die Rückführbarkeit des Katalysatorsystems hervorragende Ergebnisse erhalten werden. Sehr gute Ergebnisse werden erfindungsgemäss auch in Dioxan(en) erhalten, das (die) bis zu 30 Gew.-% Wasser und/oder bis zu 20 Gew.-% Essigsäure enthält(en).

Die folgenden Beispiele zeigen, dass erfindungsgemäss wenigstens 30 Rückführungen möglich sind, bzw. ein kontinuierlicher Betrieb über mindestens 2000 Stunden, ohne dass ein nennenswerter Umsatzrückgang oder Selektivitätsverlust und damit eine Limitierung der Laufzeit festzustellen ist. Das erfindungsgemässe Verfahren erlaubt daher erstmals die Homologisierung des Methanols in einem kontinuierlichen Verfahren mit Katalysatorrückführung durchzuführen. Die Rückführung kann gemäss dem bekannten Stand der Technik erfolgen. Hierbei kann z.B. ein Teil des Dioxans bei der destillativen Aufarbeitung abdestilliert werden und dann rückgeführt werden. Ebenso kann im Produktgemisch enthaltene Essigsäure mit dem Katalysator rückgeführt werden. Die Hydrolyse im Produktgemisch enthaltener Acetale kann in Gegenwart des Katalysators unter gleichzeitigem Abdestillieren des Acetaldehyds erfolgen.

Erfindungsgemäss wurde weiterhin gefunden, dass Umsatz und Selektivität durch Zusatz von Ethern, insbesondere von Kronenethern noch verbessert werden können.

Der Zusatz von Liganden, wie sie in Homologisierungsreaktionen bekannt sind, wie z.B. von Phosphinen, Diphosphinen, Arsinen oder Stibinen erwies sich überraschend nicht als günstig für die Rückführbarkeit.

Auch Lösungsmittel wie Anisol, Tetrahydrofuran, Diglyme oder Benzodioxan sind für den Fachmann unvorhersehbar für Homologisierungsverfahren mit Rückführung nicht geeignet.

Mit den folgenden Beispielen wird die vorliegende Erfindung näher erläutert.

Bei den angegebenen Versuchen wurde mit einem $CO/H_2$ Verhältnis von etwa 1:1 gearbeitet, das Verhältnis kann jedoch, wie bei Homologisierungen von Methanol üblich zwischen 10:1 bis 1:10 liegen, wobei ein CO-reicheres Gas in Abhängigkeit von den sonstigen Bedingungen zu einer Erhöhung des Methylacetatanteils führen kann. Auch Druck und Temperatur liegen in den üblichen Bereichen, nämlich 200 bis 350 bar und 170 bis 220°C.

Die Verweilzeiten können bei maximal 10 Stunden liegen, vorzugsweise im Bereich von 0,5 bis 3 Stunden.

Das molare Halogen/Kobalt-Verhältnis liegt, wie für Homologisierungen bekannt bei 1:1 bis 100:1, das molare Kobalt zu Methanol-Verhältnis bei 1:50 bis 1:20 000.

Im Rahmen der vorliegenden Erfindung liegen auch Systeme, die zusätzlich kleine Mengen anderer Lösungsmittel als die 1,4-Dioxane enthalten. Das Verhältnis Methanol zu Lösungsmittel ist dem Stand der Technik entsprechend, in weiten Grenzen variierbar. Jedoch wurde, um Vergleiche zu ermöglichen, in den vorliegenden Versuchen bei einem Verhältnis 1:1 gearbeitet.

*Beispiel 1*

Methanol wurde mit $CO/H_2$ im Verhältnis 1:1 bei 300 bar, 195°C und einer Verweilzeit von 2 Stunden umgesetzt. Das Verhältnis von Methanol zu 1,4-Dioxan betrug 1:1 Volumenteile. Als Katalysator wurden pro Mol Methanol 125 mg Kobaltacetat eingesetzt ($Co(OAc)_2 \times 4\ H_2O$).

Das molare Verhältnis von Co/K/J betrug 1:2:8. Methyljodid wurde in einer Menge von 426 mg pro Mol Methanol eingesetzt. Als salzartiges Jodid wurde KJ verwendet. Geringfügige während des Versuches auftretende Verluste an Methyljodid wurden jeweils ersetzt. Die Umsätze und Selektivitäten sind in Tabelle 1 wiedergegeben.

Sie wurden nach folgender Formel errechnet:

$$S\,(1) = \frac{\text{Anzahl der Mole der Komponente (1) im Reaktionsprodukt}}{\text{Mole umgesetztes } CH_3OH} \times \text{Faktor} \times 100$$

S (1) = Selektivität eines Produktes (1)
Faktor = Anzahl Mole Methanol im Produkt (1)

*Beispiel 2*

Versuch 1 wurde wiederholt, jedoch im kontinuierlichen Betrieb. Nach 2000 Stunden wurde der Versuch abgebrochen. Umsatz und Selektivität entsprachen im wesentlichen den in Beispiel 1 erhaltenen Ergebnissen.

*Beispiel 3*

Beispiel 1 wurde wiederholt, jedoch 196 mg Kaliumacetat anstelle vonKJ eingesetzt und ein molares Verhältnis von Co/K/J von 1:2:6.

*Beispiel 4*

Versuch 3 wurde wiederholt, jedoch im kontinuierlichen Betrieb. Nach 2000 Stunden wurde der Versuch abgebrochen. Umsatz und Selektivität entsprachen im wesentlichen den in Beispiel 3 erhaltenen Ergebnissen.

*Beispiel 5*

Methanol wurde, wie in Beispiel 1 beschrieben, umgesetzt, jedoch enthielt das 1,4-Dioxan 2 Gew.-% Wasser.

Der Katalysator wurde 30mal rückgeführt. Zu Beginn und Ende der Reaktion wurden Umsatz und Selektivitäten ermittelt. Wie aus Tabelle 1 hervorgeht, wurden sehr gute Ergebnisse erhalten, ohne dass eine wesentliche Änderung nach 30maliger Rückführung auftrat.

*Beispiel 6*

Beispiel 5 wurde wiederholt, jedoch im kontinuierlichen Betrieb. Nach 2000 Stunden wurde der Versuch abgebrochen. Umsatz und Selektivität entsprachen im wesentlichen den in Beispiel 5 erhaltenen Ergebnissen.

*Beispiel 7*

Methanol wurde, wie in Beispiel 1 beschrieben, umgesetzt, jedoch enthielt das 1,4-Dioxan 2 Gew.-% Essigsäure. Der Katalysator wurde 30mal rückgeführt. Zu Beginn und Ende der Reaktion wurden Umsatz und Selektivitäten ermittelt. Wie Tabelle 1 zeigt, wurden sehr gute Ergebnisse erhalten.

*Beispiel 8*

Beispiel 7 wurde wiederholt, jedoch im kontinuierliche Betrieb. Nach 2000 Stundenn wurde der Versuch abgebrochen. Tabelle 1 zeigt die Ergebnisse.

*Beispiel 9*

Methanol wurde, wie in Beispiel 1 beschrieben, umgesetzt. jedoch wurden pro Mol Methanol 145 mg $NH_4J$ und 430 mg Methyljodid eingesetzt.

Der Katalysator wurde 30mal rückgeführt. Zu Beginn und Ende der Reaktion wurden Umsatz und Selektivitäten ermittelt. Wie aus Tabelle 1 hervorgeht, traten praktisch keine Änderungen nach 30maliger Rückführung auf.

*Beispiel 10*

Versuch 9 wurde wiederholt, jedoch im kontinuierlichen Betrieb. Nach 2000 Stunden wurde der Versuch abgebrochen. Umsatz und Selektivitäten entsprachen im wesentlichen den in Beispiel 9 erhaltenen Ergebnissen.

*Beispiel 11*

Methanol wurde, wie in Beispiel 1 beschrieben, umgesetzt, jedoch wurden pro Mol Methanol 229 mg Triethylmethylammoniumjodid eingesetzt und 430 mg Methyljodid. Das Verhältnis von Methanol zu 1,4-Dioxan betrug 1:2.

Der Katalysator wurde 30mal rückgeführt. Zu Beginn und Ende der Reaktion wurden Umsatz und Selektivitäten ermittelt. Wie aus Tabelle 1 hervorgeht, traten relativ geringe Abnahmen nach 30 Stunden auf.

*Beispiel 12*

Versuch 11 wurde wiederholt, jedoch im kontinuierlichen Betrieb. Nach 2000 Stunden wurde der Versuch abgebrochen, Umsatz und Selektivitäten nahmen etwas stärker ab als in Versuch 11.

*Beispiel 13*

Methanol wurde, wie in Beispiel 1 beschrieben, umgesetzt, jedoch wurden 170 mg Kaliumjodid, 426 mg Methyljodid und 262 mg 18-Kronenether ($C_{12}H_{24}O_6$) jeweils pro Mol Methanol eingesetzt. Die Ergebnisse vor und nach 30maliger Rückführung waren etwas verbessert gegenüber Beispiel 1.

*Beispiel 14*

Beispiel 13 wurde wiederholt, jedoch im kontinuierlichen Betrieb. Nach 2000 Stunden wurde der Versuch abgebrochen. Es wurden im wesentlichen die gleichen Ergebnisse wie in Beispiel 13 erhalten.

*Beispiel 15*

Versuch 1 wurde wiederholt, jedoch als Lösungsmittel ein Gemisch methylierter 1,4-Dioxane eingesetzt. Die Ergebnisse entsprachen Versuch 1.

*Beispiel 16*

Versuch 2 wurde wiederholt, jedoch als Lösungsmittel ein Gemisch methylierter 1,4-Dioxane eingesetzt. Die Ergebnisse entsprachen Versuch 2.

*Beispiel 17*

Versuch 1 wurde wiederholt, jedoch ein Gemisch von 90 Gew.-% 1,4-Dioxan und 10 Gew.-% Chlorbenzol eingesetzt. Es wurden nur wenig schlechtere Ergebnisse erhalten als in Versuch 1.

*Beispiel 18*

Versuch 2 wurde wiederholt, jedoch ein Gemisch von 90 Gew.-% 1,4-Dioxan und 10 Gew.-% Chlorbenzol eingesetzt. Es wurden nur wenig schlechtere Ergebnisse erhalten als in Versuch 2.

*Beispiel 18a*

Beispiel 1 wurde wiederholt, jedoch ein $CO/H_2$-Verhältnis von 8:1 angewandt.

Alle Ergebnisse der Beispiele 1-18a sind in Tabelle 1 zusammengefasst, Selektivitäten sind für die Produkte Acetaldehyd, Acetaldehyddimethylacetal und Essigsäuremethylester angegeben. Weitere Produkte sind geringe Mengen an Essigsäure, Dimethylether, höhere Alkohole u.a.

TABELLE 1

| Beispiel | Promotor | Lösungsmittel | Ether | Rückführungen | Umsatz (%) | Selektivität Acetaldehyd | Acetal | Ester |
|---|---|---|---|---|---|---|---|---|
| 1 | KJ | Dioxan | — | 1 | 76 | 57,1 | 17,2 | 5,2 |
| 1 | | | — | 30 | 76,5 | 55,4 | 18,2 | 7,1 |
| 2 | | | — | 1 Std. | 80,5 | 57,1 | 17,2 | 5,3 |
| 2 | | | — | 2000 | 82 | 56,7 | 18,8 | 7,9 |
| 3 | K(OAc) | Dioxan | — | 1 | 76,2 | 57,3 | 17,1 | 5,4 |
| 3 | $CH_3J$ | | — | 30 | 76,6 | 56,7 | 18,3 | 7,5 |
| 4 | | | — | 1 | 80,4 | 57,4 | 17,1 | 5,4 |
| 4 | | | | 2000 | 81,9 | 56,9 | 18,4 | 8,0 |

TABELLE 1 (Fortsetzung)

| Beispiel | Promotor | Lösungsmittel | Ether | Rückführungen | Umsatz (%) | Selektivität Acetaldehyd | Acetal | Ester |
|---|---|---|---|---|---|---|---|---|
| 5 | KJ | Dioxan | — | 1 | 77,1 | 58,0 | 16,7 | 5,1 |
| 5 | | 12 Gew.-% | — | 30 | 77,2 | 57,5 | 16,5 | 6,5 |
| 6 | | $H_2O$ | — | 1 | 81,9 | 57,9 | 16,4 | 5,0 |
| 6 | | | — | 2000 | 82,8 | 57,3 | 16,9 | 6,9 |
| 7 | KJ | Dioxan | — | 1 | 77,7 | 58,0 | 16,4 | 7,2 |
| 7 | | 5 Gew.-% | — | 30 | 77,9 | 56,9 | 16,3 | 7,9 |
| 8 | | Essigsäure | — | 1 | 82,0 | 57,7 | 16,2 | 7,2 |
| 8 | | | — | 2000 | 82,9 | 57,4 | 16,6 | 8,9 |
| 9 | $NH_4J$ | Dioxan | — | 1 | 82 | 56,4 | 14,9 | 7,1 |
| 9 | | | — | 30 | 81,4 | 55,9 | 15,1 | 6,8 |
| 10 | | | — | 1 Std. | 83,1 | 56,4 | 14,9 | 7,1 |
| 10 | | | — | 2000 | 83,0 | 56,2 | 15,3 | 6,9 |
| 11 | $Et_3MeNJ$ | Dioxan | — | 1 | 81,6 | 58,1 | 20,2 | 7,0 |
| 11 | | | — | 30 | 80,1 | 54,8 | 19,1 | 6,2 |
| 12 | | | — | 1 Std. | 81,6 | 58,1 | 20,2 | 7,0 |
| 12 | | | — | 2000 | 80,4 | 55,2 | 19,2 | 6,4 |
| 13 | KJ | Dioxan | 18-Kronen-ether | 1 | 90,2 | 58,3 | 20,3 | 7,2 |
| 13 | | | » | 30 | 88,4 | 54,9 | 18,9 | 6,4 |
| 14 | | | » | 1 Std. | 90,2 | 58,3 | 20,3 | 7,2 |
| 14 | | | » | 2000 | 88,7 | 55,0 | 19,2 | 6,7 |
| 15 | KJ | Gemisch | — | 1 | 76 | 57,1 | 17,2 | 5.3 |
| 15 | | methy- | — | 30 | 76,5 | 55,4 | 18,2 | 7,1 |
| 16 | | lierter | — | 1 Std. | 80,5 | 57,1 | 17,2 | 5,3 |
| 16 | | 1,4-Dioxane | — | 2000 | 82 | 56,7 | 18,8 | 7,9 |
| 17 | KJ | Dioxan | — | 1 | 75,8 | 57,0 | 17,1 | 5,3 |
| 17 | | + 10% | — | 30 | 76,0 | 54,0 | 17,6 | 6,1 |
| 18 | | Chlor- | — | 1 Std. | 75,8 | 57,0 | 17,1 | 5,3 |
| 18 | | benzol | — | 2000 | 80,7 | 55,2 | 18,0 | 6,8 |
| 18a | KJ | Dioxan | — | 1 | 71 | 15,0 | 6,9 | 51,1 |
| 18a | KJ | Dioxan | — | 30 | 72,1 | 14,4 | 6,7 | 52,9 |

In Tabelle 2 sind Ergebnisse von Versuchen gemäss Beispiel 2 zusammengestellt, bei denen jedoch andere für Homologisierungen gemäss dem Stand der Technik übliche Lösungsmittel eingesetzt wurden. Nach 6 Stunden wurden die Versuche abgebrochen, mit Ausnahme von Versuch 19 mit Diglyme, der nach 20 Stunden abgebrochen wurde.

Die Ergebnisse machen deutlich, dass die Lösungsmittel der Versuche 19-26 weniger gut geeignet sind für Homologisierungen mit Rückführung als die erfindungsgemässen Lösungsmittel. Es wurden sowohl deutliche Umsatzrückgänge als auch Selektivitäten festgestellt. Ferner wurden in allen Versuchen mit Ausnahme des Versuchs 22 Zersetzungsprodukte der Lösungsmittel gefunden.

Das Verhältnis von Methanol zu Lösungsmittel betrug in allen Beispielen 1 Vol.-Teil : 1 Vol.-Teil.

TABELLE 2

| Beispiel | Promotor | Lösungsmittel | kontinuierlich | Umsatz (%) | Selektivität Acetaldehyd | Acetal | Ester | Zersetzungsprodukte |
|---|---|---|---|---|---|---|---|---|
| 19 | KJ | Diglyme | 1 Std. | 80,5 | 53,8 | 13,6 | 17,3 | höher molekulare |
| 19 | | | 6 Std. | 79,0 | 43,3 | 9,2 | 32,2 | Ether |
| 19 | | | 20 Std. | 58,6 | 23,7 | 15,3 | 46,7 | |

TABELLE 2 (Fortsetzung)

| Beispiel | Promotor | Lösungsmittel | kontinuierlich | Umsatz (%) | Selektivität Acetaldehyd | Acetal | Ester | Zersetzungsprodukte |
|---|---|---|---|---|---|---|---|---|
| 20 | KJ | Tetraglyme | 1 Std. | 81,1 | 49,7 | 11,9 | 20.9 | höher molekulare |
| 20 | | | 6 Std. | 75,5 | 34,9 | 19,8 | 41,8 | Ether |
| 21 | KJ | Diphyl | 1 Std. | 74,2 | 18,0 | 60,4 | 9,0 | nicht identifizierte |
| 21 | | | 6 Std. | 63,0 | 13,1 | 20,3 | 7,9 | hochmolekulare Prod. |
| 22 | KJ | Toluol | 1 Std. | 74,3 | 21,3 | 61,1 | 4,0 | — |
| 22 | | | 6 Std. | 64,4 | 17,9 | 23,2 | 4,1 | |
| 23 | KJ | Sulfolan | 1 Std. | 63,0 | 18,0 | 13,1 | 36,4 | nicht identifizierte |
| 23 | | | 6 Std. | 59,7 | 16,9 | 11,7 | 29,4 | Schwefel enthaltende höher molekulare Prod. |
| 24 | KJ | Tetrahy- | 1 Std. | 78,4 | 65,2 | 11,4 | 7,2 | höher molekulare |
| 24 | | drofuran | 6 Std. | 65,6 | 49,1 | 10,0 | 6,5 | Ether |
| 25 | KJ | Anisol | 1 Std. | 78,7 | 45,7 | 30,0 | 5,8 | Phenol |
| 25 | | | 6 Std. | 64,8 | 39,6 | 26,2 | 4,6 | |
| 26 | KJ | Benzo- | 1 Std. | 70,1 | 53,8 | 22,9 | 7,0 | höher molekulare |
| 26 | | dioxan | 6 Std. | 60,8 | 45,3 | 18,7 | 4,9 | Ether |

Tabelle 3 enthält Ergebnisse der Versuche 27-30, in denen als Promotor Triphenylphosphin (TPP) und verschiedene Lösungsmittel eingesetzt wurden.

In allen Versuchen wurden pro Mol Methanol 125 mg Kobaltacetat, 426 mg Methyljodid und 262 mg Triphenylphosphin eingesetzt. Die Versuche wurden im übrigen entsprechend Beispiel 2 durchgeführt.

Ausser den angegebenen aus den Lösungsmitteln stammenden Zersetzungsprodukten wurde in allen Fällen die Bildung von Triphenylphosphinoxid unter Abnahme des Triphenylphosphins bzw. Phosphoniumjodids beobachtet.

Die Versuche der Tabelle 3 machen deutlich, dass die Gegenwart des Phosphins zu schlechteren Ergebnissen führt, auch im Falle der Verwendung von 1,4-Dioxan als Lösungsmittel. Schlechtere Ergebnisse, die jedoch nicht wiedergegeben sind, werden auch erhalten, wenn man KJ bzw. NaJ oder die offenbarten Ammoniumjodide als Promotor teilweise durch Triphenylphosphin ersetzt.

TABELLE 3

| Beispiel | Promotor | Lösungsmittel | kontinuierlich | Umsatz (%) | Selektivität Acetaldehyd | Acetal | Ester | Zersetzungsprodukte |
|---|---|---|---|---|---|---|---|---|
| 27 | TPP | Dioxan | 1 Std. | 77,1 | 61 | 14 | 5 | — |
| 27 | | | 6 Std. | 74,6 | 55,5 | 16,5 | 10,7 | |
| 27 | | | 20 Std. | 72,4 | 44,1 | 13,2 | 17,5 | |
| 28 | TPP | Tetrahy- | 1 Std. | 77,0 | 65,0 | 10,1 | 6,0 | höher molekulare |
| 28 | | drofuran | 6 Std. | 66,4 | 51,2 | 8,9 | 6,9 | Ether |
| 29 | TPP | Anisol | 1 Std. | 77 | 45,0 | 29,2 | 4,2 | Phenol |
| 29 | | | 6 Std. | 63,9 | 41,4 | 26,3 | 4,4 | |
| 30 | TPP | Toluol | 1 Std. | 80 | 39,0 | 24,0 | 2,0 | — |
| 30 | | | 6 Std. | 67,7 | 24,2 | 20,9 | 2,2 | |

**Patentansprüche**

1. Verfahren zur Homologisierung von Methanol, dadurch gekennzeichnet, dass die Homologisierung unter Verwendung des Katalysatorsystems enthaltend:

a) Kobalt und/oder (eine) Kobaltverbindung(en),

b) wenigstens ein Jodid aus der Gruppe: LiJ, NaJ,

KJ, RbJ, CsJ, (N $R_1R_2R_3R_4$) $^+J^-$, und/oder wenigstens eines dieser Jodide bildende Verbindungen, wobei $R_1$, $R_2$, $R_3$ und $R_4$ H, Alkyl- und Arylgruppen, bevorzugt H, $CH_3$, $C_2H_5$ und $C_6H_5$ sein können,

c) Methyljodid und/oder Methyljodid bildende Verbindungen

in Gegenwart von 1,4-Dioxan und/oder alkyliertem 1,4-Dioxan durchgeführt wird und dass die Homologisierung kontinuierlich unter Rückführung des Katalysatorsystems durchgeführt wird.

2. Verfahren nach Anspruch 1, dadurch gekennzeichnet, dass das 1,4-Dioxan und/oder alkylierte Dioxan bis zu 30 Gew.-% Wasser und/oder bis zu 20 Gew.-% Essigsäure enthält.

3. Verfahren nach den Ansprüchen 1 und 2, dadurch gekennzeichnet, dass das salzartige Jodid wenigstens eine Verbindung aus der Gruppe KJ, NaJ, $NH_4^+J^-$ und N $(CH_3)_4^+J^-$ ist.

4. Verfahren nach den Ansprüchen 1 bis 3, dadurch gekennzeichnet, dass das Katalysatorsystem zusätzliche Ether, bevorzugt Kronenether enthält.

5. Verfahren nach den Ansprüchen 1 bis 4, dadurch gekennzeichnet, dass in Gegenwart des Homologisierungskatalysators die im Produktgemisch enthaltenen Acetale hydrolysiert werden unter gleichzeitiger destillativer Abtrennung des Acetaldehyds.

6. Verfahren nach den Ansprüchen 1 bis 5, dadurch gekennzeichnet, dass das rückgeführte Katalysatorsystem Wasser, Essigsäure, höhere Homologisierungsprodukte und Dioxan(e) enthalten kann.

## Claims

1. Process for the hydroformylation of methanol, characterized in that the reaction is carried out in the presence of a catalyst comprising:

a) cobalt and/or (a) cobalt compound(s),

b) at least one iodide of the group: LiJ, NaJ, KJ, RbJ, CsJ, (N $R_1R_2R_3R_4$) $^+J^-$, and/or compounds, which form at least one iodide of this group, whereby $R_1$, $R_2$, $R_3$ and $R_4$ may be H, alkyl- and aryl group, preferably H, $CH_3$, $C_2H_5$ and $C_6H_5$,

c) methyl iodide and/or methyl iodide forming compounds

in the presence of 1,4-dioxane and/or alkylated 1,4-dioxane and that the hydroformylation is carried out continuously and the catalyst is recycled.

2. Process according to claim 1, characterized in that the 1,4-dioxane and/or alkylated 1,4-dioxane contain up to 30 weight-% of water and/or 20 weight-% of acetic acid.

3. Process according to claims 1 and 2, characterized in that the saltlike iodide is at least one compound of the group KJ, NaJ, $NH_4^+J^-$ and N $(CH_3)_4^+J^-$.

4. Process according to claims 1 to 3, characterized in that the catalyst contains additionally ethers, preferably crown ethers.

5. Process according to claims 1 to 4, characterized in that the acetales in the product mixture are hydrolized in the presence of the hydroformylation catalyst, whereby simultaneously acetic aldehyde is separated by distillation.

6. Process according to claims 1 to 5, characterized in that the catalyst reclyled, may contain water, acetic acid, higher hydroformylation products and dioxane(s).

## Revendications

1. Procédé de carbonylation du méthanol en présence d'hydrogène, caractérisé en ce que l'on opère en présence d'un catalyseur comprenant:

a) cobalt et/ou un (des) composé(s) du cobalt,

b) au moins un iodure ionique choisi dans le groupe constitué par LiJ, NaJ, KJ, RbJ, CsJ, (N $R_1R_2R_3R_4$) $^+J^-$ et/ou des composés qui forment au moins un composé de cette groupe, où $R_1$, $R_2$, $R_3$ et $R_4$ peuvent être H, alcoyle et aryle, préférable H, $CH_3$, $C_2H_5$ et $C_6H_5$,

c) l'iodure de méthyle et/ou des composés, qui forment l'iodure de méthyle

et en présence de dioxanne-1,4 et/ou dioxanne-1,4 alcoylé et que l'on opère continu avec recyclage du catalyseur.

2. Procédé selon la revendication 1, caractérisé en ce que le dioxanne-1,4 et/ou le dioxanne-1,4 alcoylé contient jusqu'à 30% en poids de l'eau et/ou jusqu'à 20% en poids de l'acide acétique.

3. Procédé selon les revendications 1 et 2, caractérisé en ce que l'iodure ionique est au moins un composé choisi dans le groupe KJ, NaJ, $NH_4^+J^-$ et N $(CH_3)_4^+J^-$.

4. Procédé selon les revendications 1 à 3, caractérisé en ce que le catalyseur contient additionnel des éthers, préférable des éthers à couronne.

5. Procédé selon les revendications 1 à 4, caractérisé en ce que les acétals compris dans le mélange produit sont hydrolysés en présence du catalyseur selon la revendication avec séparation en même temps de l'aldehyde acétique par distillation.

6. Procédé selon les revendications 1 à 5, caractérisé en ce que le catalyseur recyclé peut contenir $H_2O$, $CH_3COOH$, dioxanne-1,4 et des produits de la carbonylation du méthanol d'un degré supérieur.